# EUROPEAN PATENT APPLICATION

(11) **EP 4 266 056 A1**
(43) Date of publication of application: **25.10.2023**
(21) Application number: 22169328.6
(22) Date of filing: 21.04.2022
(51) Int. Cl.: G01N 33/543, G01N 33/551, G01N 33/554, G01N 33/569

(54) **SIZE-BASED DETECTION AND QUANTIFICATION OF FUNCTIONAL BIO-NANOPARTICLES**

(71) Applicant: Athanor Biosciences, Inc., Halethorpe, Maryland 21227 (US)
(72) Inventor: LEBOWITZ, Michael S., Halethorpe, 21227 (US); GHANBARI, Hossein A., Halethorpe, 21227 (US)
(74) Representative: Canzler & Bergmeier Patentanwälte Partnerschaft mbB

(57) **Abstract**

The invention relates to a method for detecting a bio-nanoparticle comprising: contacting a test sample comprising a bio-nanoparticle having a size dimension of about 50 nanometers or greater with a detection nanoparticle, the detection nanoparticle having a size dimension of about 50 nanometers or greater, the detection nanoparticle comprising a plurality of binding agents at a surface of the nanoparticle; wherein upon the contact, the detection nanoparticle binds a plurality of the bio-nanoparticle via a specific binding between one of the binding agents and a binding partner of each of the plurality of the bio-nanoparticles, the binding forming a multi-nanoparticle complex that includes multiple bio-nanoparticles and optionally includes multiple detection nanoparticles in a three dimensional complex; and detecting the multi-nanoparticle complex according to a size-detection regime.

## Description

### Cross Reference to Related Application

This application claims filing benefit of United States Provisional Patent Application Serial Number 63/094,455, having a filing date of October 21, 2020, entitled "Sized-Based Detection and Quantification of Functional Bio-Nanoparticles (BNPs)," which is incorporated herein by reference in its entirety.

### Background

Currently viruses and other bio-nanoparticles (BNPs) are detected and quantified using a very small sub-unit specific to the BNP in question without any regard to status of the entity itself. For instance, SARS-CoV-2 is detected by targeting a very small segment of its unique RNA or a polypeptide specific to the virus, and detection of such targets may provide only detection of degradation remains of the virus, rather than detection of the complete, functional virus. For example, detection of SARS-CoV-2 RNA in sewage by current detection techniques may not correlate to detection of any intact virus and may be merely the identification of harmless fragments of the virus.

Thus, a need exists for detection of BNPs that can ensure the complete particle is being detected, rather than just a fragment of the particle, e.g., detection of complete virus, phage, exosome, etc. Detection techniques that can differentiate between whole BNP and fragments thereof could provide increased sensitivity and specificity in a variety of applications, including infectious disease detection (both in individual diagnosis and environmental, e.g., public health, detection) as well as cancer and other disease detection.

### Summary

According to one embodiment, disclosed are methods for detecting a bio-nanoparticle (BNP). A method can include contacting a sample with a detection nanoparticle. The detection nanoparticle has an average size dimension of about 50 nm or larger and the detection nanoparticle also includes a plurality of binding agents at a surface of the detection nanoparticle. Upon the contact, the detection nanoparticle can bind a plurality of the BNP of interest via the binding agents, the binding forming a multi-nanoparticle complex that includes multiple bio-nanoparticles and optionally includes multiple detection nanoparticles in a three-dimensional complex. The BNP of interest can generally have a size dimension of about 50 nm or larger, and thus, the multi-nanoparticle complex can have a much larger dimension than the nanoparticles forming the complex, e.g., about 200 nm or greater, or even larger, about 300 nm or greater or about 400 nm or larger, such as up to about 1 micrometer, or about 2 micrometers in some embodiments. A method can also include detecting the multi-nanoparticle complex according to a size-detection regime.

Methods disclosed herein can be utilized in disease diagnosis, prognosis, and/or companion diagnostics, as well as in BNP tracking, for instance in public health applications.

### Brief Description of the Figures

A full and enabling disclosure of the present subject matter, including the best mode thereof to one of ordinary skill in the art, is set forth more particularly in the remainder of the specification, including reference to the accompanying figures in which:
FIG. 1 schematically illustrates a bacteriophage as may be utilized in embodiments of disclosed methods.
FIG. 2 depicts dot blot results showing formation of modified phage detection particles as described herein.
FIG. 3 graphically illustrates particle counts for detection particles, target BNPs, and multi-particle complexes formed including the two.

Repeat use of reference characters in the present specification and drawings is intended to represent the same or analogous features or elements of the present invention.

### Detailed Description

Reference will now be made in detail to various embodiments of the disclosed subject matter, one or more examples of which are set forth below. Each embodiment is provided by way of explanation of the subject matter, not limitation thereof. In fact, it will be apparent to those skilled in the art that various modifications and variations may be made in the present disclosure without departing from the scope or spirit of the subject matter. For instance, features illustrated or described as part of one embodiment, may be used in another embodiment to yield a still further embodiment.

In general, disclosed herein are sized-based detection techniques for detection of BNPs and detection nanoparticles that can be utilized in the techniques. Detection nanoparticles disclosed herein can target a functional BNP with high specificity through specific binding of one or more entities unique to the functional BNP of interest. In some embodiments, a detection nanoparticle can target an entity of a BNP that is relevant to its function, and as such, the methods can provide improvements in detection of complete and functional BNPs.

Upon contact, e.g., incubation of a detection nanoparticle with a test sample that includes the BNP of interest and subsequent binding of the two, a significantly larger multi-nanoparticle complex is formed. Formation of this larger complex can be detected by the large size, as it is much larger than either of the nanoparticles forming the newly formed complex. Smaller particles, including unbound detection particles and/or detection particles bound to fragments of the BNP of interest rather than the complete BNP can be excluded based on the size difference. Beneficially, the method can be relatively simple and inexpensive, as the process can be carried out with few or no sample preparation steps and the detection based on sizing can take minutes rather than hours or days, even in those cases in which several replicates are carried out to improve accuracy.

Detection particles can be either synthetic or biologic in origin, and in general, can encompass any nano-sized particle to which a specific binding agent can be attached. A detection particle can generally have a size (e.g., an average size) of about 50 nm or greater, such as about 75 nm or greater or about 100 nm or greater, and about 300 nm or less, such as about 250 nm or less, or about 200 nm or less, in some embodiments.

In one embodiment, a detection particle can be a bacteriophage that has been modified to include one or more specific binding agents at a surface that exhibit specific binding with a BNP of interest. Bacteriophage (or more simply phage) are viruses that infect bacterial cells. These viruses consist of a protein coat which encapsulates a DNA or RNA genome. When phage infect a bacterial cell, they can co-opt the host bacterial system to produce large numbers of phage copies and ultimately lyse the bacterial cell, releasing the new phage to the surrounding environment. Bacteriophage can be beneficial in some embodiments, as they can be engineered to display many copies of a protein (e.g., a specific binding agent) on the surface of the phage. This can be beneficial as when two relatively large entities interact, i.e., a detection nanoparticle and a BNP of interest, the existence of multiple engagement points can greatly enhance the overall strength of the interaction (avidity). By way of example, when considering bacteriophage λ, the displayed specific binding agent(s) can be ligated to the phage gpD coat protein. 400 copies of the gpD protein are used by the phage to construct its coat and as such up to 400 copies of the specific binding agent(s) can be displayed on the phage surface.

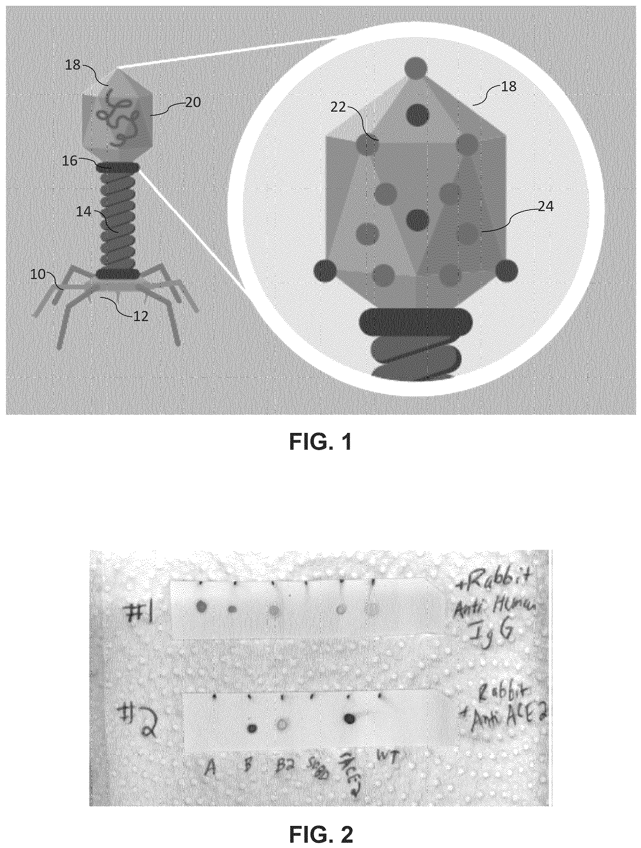

In one embodiment, a detection nanoparticle can be a multivalent particle, e.g., a multivalent bacteriophage, and can include multiple different specific binding agents at the surface of the particle. FIG. 1 schematically illustrates one embodiment of a multivalent bacteriophage. As illustrated, a multivalent bacteriophage can include typical bacteriophage components including tail fiber 10, spikes 12, and a sheath 14. A collar 16 typically separates the sheath 14 from the capsid head 18, which encases the bacteriophage DNA 20. The capsid head 18 is formed from a plurality of coat proteins, e.g., gpD, gpE and gpC coat protein in the case of bacteriophage λ. Multivalent bacteriophage can include two or more specific binding agents 22, 24 on the capsid head 18 that each specifically bind different targets of a single BNP of interest (either different proteins or different binding sites of a single protein or that each specifically bind different targets of different BNP, i.e., a single detection nanoparticle can be utilized in detection of multiple different BNP.

The ability to display large copy numbers of polypeptides on the surface of the detection nanoparticle allows for the construction of binding entities that can be highly multivalent and that can bind with much greater binding avidity as compared to binders exhibiting lower valency binding, e.g., monovalent or bivalent binders. Furthermore, the ability to display multiple different binding entities simultaneously on the surface of the detection nanoparticle allows for the ability to interact with the target in multiple ways. These "Super-Binders" can display hundreds of copies of one or more specific binding partners and as such can bind to exosomes, viruses or other BNP targets with extremely high avidity.

The use of phage as detection particles can be advantageous in some embodiments as phage can be relatively simple to genetically engineer and are easy to purify. Furthermore, phage can be produced at extremely high-yield in readily available bio-fermenters. As such, the development and manufacture processes can be rapid and highly cost-effective.

However, it will be understood by those skilled in the art that detection nanoparticles are not limited to those based on bacteriophage, and beads, spheres, particles, or granules of other biologic origin, as well as synthetic particles are encompassed herein.

Synthetic detection particles can be produced from a number of materials which will be apparent to one skilled in the art according to known formation processes including, without limitation, metals, ceramics, or polymer material. Synthetic detection nanoparticle materials may include gold, silver, cobalt, nickel, platinum, metal oxides, etc. Exemplary nanoparticles can include, without limitation, Au, Ag, Ni, ZnS, ZnO, TiO₂, SiO₂, latex, polysaccharides, polystyrenes, etc.

Various types of metallic nanoparticles can be utilized in forming detection nanoparticles including, but not limited to, gold particles, silver particles, copper particles, platinum particles, cadmium particles, composite particles (e.g., silver and gold or copper and silver), and gold hollow spheres. Metal nanoshells can include a core and a metallic coating having a defined core radius to shell thickness ratio. The core can be formed of any of a variety of materials including metals, oxides, and polymers. Suitable metals for the shell can include gold, silver, copper, platinum, palladium, lead, and iron.

Specific binding agents can be attached at a surface of a detection nanoparticle according to any suitable attachment mechanism. In one embodiment, one or more specific binding agents can be attached to a detection nanoparticle through utilization of a linking agent. For instance, when considering a bacteriophage as a detection nanoparticle, a transformed bacteriophage can be utilized, which has been transformed to express a linking peptide that includes a particular amino acid or amino acid sequence, e.g., including lysine or cysteine, that can then be utilized as a linking agent for attachment of a specific binding agent at a surface of the bacteriophage. For instance, a linking agent including a cysteine (C) residue can be utilized to attach a binding agent to the detection nanoparticle by means of its thiol and an amino group of the binding agent (e.g., the N-terminus of a peptidic binding agent) and a linking agent including a lysine (L) residue can be utilized to attach a binding agent to the detection nanoparticle by means of its amino group and a carboxyl group of the binding agent (e.g., the C-terminus of a peptidic binding agent).

Formation methods of such a bacteriophage-based detection nanoparticle can include transfecting a bacterial cell with a bacteriophage and also with an expression plasmid that includes a nucleic acid sequence encoding a linking peptide in a fashion that can be expressed by the bacteriophage. For example, the expression plasmid can encode a fusion phage coat protein that encodes the linking peptide in conjunction with a coat protein of the bacteriophage.

An expression plasmid can be produced by recombinant DNA technology as known. A fusion coat protein encoded by the expression plasmid can include a native or wild type phage coat protein with an added N- or C-terminal extension that can be directly or indirectly fused to a terminus of the coat protein that encodes a peptide including the linking agent (e.g., indirectly fused by inclusion of a spacer between the two). An expression plasmid can include a DNA sequence encoding a phage coat protein ligated to a DNA sequence encoding the linking peptide such that the exogenous polypeptide sequence(s) is in frame with the coat protein sequence. DNA encoding a short linker sequence may be placed between the sequences if desired, for instance, to achieve successful expression.

An expression plasmid can be transfected into the bacterial cell prior to or in conjunction with infection of the bacterial cell with a phage that naturally includes the coat protein of the fusion coat protein encoded by the expression plasmid. The coat protein encoded in the expression plasmid and expressed with an exogenous linking polypeptide as a fusion coat protein can vary depending upon the phage type. The phage may be any bacteriophage known to those skilled in the art, including but not limited to λ, M13, T4, T7, ϕX174.

By way of example, when forming a detection nanoparticle based on a λ phage, an expression plasmid can include DNA encoding one or more fusion coat proteins based on one or more of the gpD, gpE or gpC coat proteins in conjunction with the encoding of one or more exogenous linking polypeptides in any combination. For example DNA of one or more plasmids can encode a first linking peptide in conjunction with a gpD coat protein as well as encoding that same linking peptide in conjunction with a gpE coat protein, DNA of one or more plasmids can encode a first linking peptide in conjunction with a gpD coat protein as well as a second, different linking peptide in conjunction with a gpD coat protein, can encode a first linking peptide in conjunction with a gpD coat protein as well as a second, different linking peptide in conjunction with a different, e.g., gpE, coat protein, or any combination thereof.

If an engineered M13 phage-based detection nanoparticle is to be formed, one or more of the pVIII, pill, pVI, pVII or pIX proteins can generally be encoded in an expression plasmid in conjunction with one or more linking polypeptides. Similarly, the gp23 and/or gp24 proteins can generally be encoded when forming a modified T4 bacteriophage and the gp10A and/or gp10B proteins can be encoded in an expression plasmid when forming a modified T7 phage. For phage ϕX174, the gpF and/or gpG proteins can generally be encoded in conjunction with one or more linking polypeptides.

A hybrid DNA sequence encoding a fusion coat protein can be placed into a bacterial expression plasmid under the control of a suitable bacterial expression promoter. A promoter can be an inducible promoter, a copy of a native phage promoter or any promoter deemed appropriate by one skilled in the art. The expression plasmid can be one that provides for transient expression of the fused coat protein in the bacterial cell. Transient expression systems have been used as tools of recombinant technology for many years and as such is not described in detail herein. By way of example and without limitation, suitable transient expression systems can include the pETDuet^{™} family of vectors from Novagen^{®}/EMD Millipore.

In forming a multivalent bacteriophage, in one embodiment, a single expression plasmid can include multiple different hybrid DNA sequences, each of which encode a different fusion coat protein. For instance, in one embodiment, an expression plasmid can include one or more variant copies of a hybrid DNA sequence, each of which encode a different linking polypeptide extension of the same base coat protein. The different exogenous linking sequences can thus be designed to attach a different specific binding agent to the surface of the bacteriophage. In another embodiment, multiple different plasmids may be used in forming a multivalent bacteriophage, with different plasmids including different hybrid DNA sequences that encode for a different fusion coat protein (e.g., different by the linking polypeptide extension, the phage coat protein, or both).

In another embodiment, detection nanoparticle bacteriophage may display only a single linking polypeptide sequence, but may include multiple different fusion coat proteins, with the linking polypeptide sequence as a component of different fusion coat proteins that incorporate different types of base coat proteins of the native phage.

When using different expression plasmids to carry different fusion coat protein DNA, the regulatory components of the expression plasmids can be the same or differ from one another. For instance, in one embodiment, different expression plasmids can be essentially the same as one another other than the fusion coat protein DNA sequences. In one embodiment, different selection markers can be incorporated on the different expression plasmids, which can be used to ensure that selected production bacteria have incorporated all plasmid types. In one embodiment, different plasmids or different expression components of a single plasmid can incorporate different promotors driving expression of the fusion coat proteins, for instance, different strength promoters, thus allowing for the fusion coat proteins with different linking polypeptide extensions to be produced at varying levels which can also allow for incorporation of the different fusion coat proteins into a modified phage at different ratios.

DNA sequences incorporated in an expression plasmid can encode a linking polypeptide of any length. For instance, a DNA sequence of an expression plasmid can encode a linking polypeptide that is about 20 amino acids or fewer in length, for instance about 15 amino acids or fewer, about 10 amino acids or fewer, about 5 amino acids or fewer in some embodiments, though longer linking polypeptides are also encompassed herein. In general, a linking polypeptide of a fusion coat protein can be of any length provided it does not interfere with the incorporation of the fusion coat protein in the bacteriophage during formation thereof by the bacterial cell.

Upon development of one or more expression plasmids that include DNA encoding the one or more linking peptides, the plasmid(s) can be transfected into a host bacterial cell. The host bacterial cell can be any suitable type that is also infectable by the phage that is to be the basis for the engineered phage product. For instance, when forming an engineered bacteriophage λ, the host bacterial cell can be an E. *coli* and an E. *coli* can thus be transfected with the expression plasmid(s) according to standard transfection practice. Suitable bacterial hosts for phage infection are known to those in the art.

In conjunction with or subsequent to the transfection of the host with the one or more expression plasmid(s), the host can be infected with the phage of choice. Depending upon the transfection/expression system utilized, additional components as necessary can be supplied to the bacterial host. For instance, if an inducible promoter is incorporated in the expression plasmid(s), the inducing agent can also be supplied to the bacterial host during phage infection.

Upon transfection and infection, the bacterial host can produce the modified bacteriophage that exhibits the linking peptides at a surface. The amount of linking peptide incorporated into a modified bacteriophage can be controlled, for instance through selection of the promoter strength of an expression plasmid. Such an approach can be used to control relative amount of different linking peptides in a bacteriophage as well as relative amount of the natural, e.g., wild type, coat protein vs. the fusion coat protein. In such an embodiment, the natural phage coat protein upon which the fusion coat protein is based can be maintained to a controlled extent on the engineered phage. Thus, the engineered phage can include a portion of the coat protein lacking any fused linking polypeptide in addition to the fused coat protein.

In one embodiment, the bacterial cell can be infected with a knockout phage in which the wild-type coat protein expression has been silenced or deleted. In this case, all of the coat protein of the type incorporated in the expression plasmid (e.g., all gpD coat protein of a bacteriophage λ) can be present in the expressed engineered phage as fusion coat protein exhibiting a linking peptide.

Following transfection and infection, a host bacteria can be grown until lysis of the bacteria. Once bacterial cell lysis has occurred, the phage can be purified and characterized using standard techniques. It should be noted that loss of infectivity by the modified phage is not a problem for the use of the modified phage. Modified phage as described can thus serve as detection nanoparticles that are easily manufactured in bacterial cultures and that can be grown at large scale in standard bio-fermenters.

Following lysis, modified bacteriophage may be purified by any number of methods known to those skilled in the art for bacteriophage purification. These methods include, but are not limited to, polyethylene glycol (PEG) precipitation, tangential flow filtration, affinity chromatography, etc. Modified bacteriophage may be further concentrated, devoided of bacterial endotoxins, and characterized by standard methods known to those skilled in the art.

Following formation, the modified bacteriophage can be contacted with the binding agent(s) of choice under conditions to effect attachment of the binding agent(s) to the bacteriophage via the linking agents.

Methods of coupling synthetic nanoparticles to one or more binding agent(s) in formation of a synthetic detection nanoparticle are known in the art. One such method is by passive adsorption. This method involves adjusting the pH of a solution containing the detection nanoparticle (e.g., a metal nanoparticle) to a pH at which the binding agent has a positive charge, mixing a nanoparticle-containing mixture with the binding agent solution, and centrifuging the resultant mixture. The detection nanoparticle including the binding agent at a surface is then obtained by removing the supernatant and resolubilizing the precipitate.

A binding agent can be coupled to a synthetic nanoparticle either directly or indirectly through utilization of a linking agent. For instance, a direct reaction can be utilized in which an activated group either on the detection nanoparticle or on the binding agent can react with a functional group on either the binding agent or on the detection nanoparticle. An indirect approach can be utilized in which a heterobifunctional linking agent can be initially reacted with either the nanoparticle or the binding agent followed by reaction with the other component.

For example, a large number of heterobifunctional compounds are available for linking to entities. Illustrative linking agents include: azidobenzoyl hydrazide, N-[4-(p-azidosalicylamino)butyl]-3'-[2'-pyridyldithio]propionamide), bis-sulfosuccinimidyl suberate, dimethyladipimidate, disuccinimidyltartrate, N-y-maleimidobutyryloxysuccinimide ester, N-hydroxy sulfosuccinimidyl-4-azidobenzoate, N-succinimidyl [4-azidophenyl]-1,3'-dithiopropionate, N-succinimidyl [4-iodoacetyl]aminobenzoate, glutaraldehyde, succinimidyl-[(N-maleimidopropionamido) polyethyleneglycol] esters (NHS-PEG-MAL), succinimidyl 4-[N-maleimidomethyl]cyclohexane-1-carboxylate, 3-(2-pyridyldithio)propionic acid N-hydroxysuccinimide ester (SPDP), and 4-(N-maleimidomethyl)-cyclohexane-1-carboxylic acid N-hydroxysuccinimide ester (SMCC).

In some embodiments, a nanoparticle can include polyethylene glycol (PEG) at a surface that can be utilized for attachment to a binding agent or to a linking agent, which can then be further reacted with a binding agent. A synthetic nanoparticle can include PEG at a surface upon formation or can be processed following formation according to known PEG-ylation techniques to provide the polymer at a surface of the particle. Further reaction of the PEG polymer can be utilized for attachment of a binding agent (direct attachment) or a linking agent (indirect attachment) to the synthetic nanoparticle through activation of the PEG with maleimides, vinyl sulfones, pyridyl disulfides, or other compounds reactive to thiols of the binding/linking agent, thus forming a polymer-protein conjugate upon this reaction. In one embodiment, a PEG-protein conjugate can maximize the selectivity of a further PEG conjugation to the N-terminus of an unprotected polypeptide chain by taking advantage of the differences between pKa values of the α-amino group of the N-terminal amino acid residue and the ε amino group of Lys residues in a peptide backbone conjugated to the PEG.

A binding agent of a detection nanoparticle can be any compound that can selectively target and bind an entity of a BNP of interest. In some embodiments, the specific binding agent of the detection nanoparticle can be selected from one or more of the following: nucleic acid, a nucleoprotein, an antibody, a polypeptide, a protein, a receptor or a target molecule, an aptamer, a saccharide, a polysaccharide, a glycopeptide, a lipid, a lipoprotein. Binding agents can include, but are not limited to, antibodies or active fragments thereof (forming an antibody/epitope complex), antigens, nucleic acids (e.g., natural or synthetic DNA, RNA, GDNA, HDNA, cDNA, mRNA, tRNA, etc.), lectins, sugars (e.g., forming a lectin/sugar complex), glycoproteins, receptors and their cognate ligand (e.g., growth factors and their associated receptors, cytokines and their associated receptors, signaling receptors, etc.), cell membrane constituents and associated structures, and combinations thereof.

Specific binding agents can target various different proteinaceous targets of a BNP, e.g., a viral BNP. Multiple binding agents of a single detection nanoparticle can target the same or different proteins of a BNP. For instance, the binding agent(s) can incorporate a natural protein binding partner of a surface protein of a BNP pathogen or a mimic thereof. For example, to engage a receptor of a BNP, a natural ligand can be employed as a specific binding agent of a detection nanoparticle or in the reverse to engage a ligand a mimic of the receptor can be employed as a specific binding agent of a detection nanoparticle.

In some embodiments, a binding agent can be derived from known antibodies produced in individuals previously known to have had immune responses to a targeted viral BNP, where this information is available. Binding agent sequences may bind to a single strain, multiple strains of a certain family and/or different families of pathogens, e.g., viral pathogens.

In one embodiment, different binding agents of a multi-valent detection nanoparticle can specifically bind a single protein target, e.g., different peptide sequences of a single protein of a BNP. For example, a first binding agent specific for a first targeted protein of a BNP (e.g., a first binding site of a viral coat protein) and a second binding agent specific for a second location of the same protein (e.g., a second binding site of the same viral coat protein).

In one embodiment, different binding agents of a detection nanoparticle can specifically bind different proteins of the same BNP. For instance, a first binding agent can specifically bind a first coat protein of a viral BNP and a second binding agent can bind a different coat protein of the same viral BNP. Of course, any combination of binding agents are encompassed herein as well.

In one embodiment, a binding agent can be developed from a protein of a BNP. For instance, a binding agent can include one or more single-chain antibodies (scFv) and/or mimics of cellular receptors of viral coat proteins. For example, a binding agent can be identical to (e.g., correspond to) a known antibody of a BNP protein or a binding fragment thereof or can be a functional mutant or homologue of an antibody or an antibody fragment.

As utilized herein, the term "homologue" generally refers to a nucleotide or polypeptide sequence that differs from a reference sequence by modification(s) that do not affect the overall functioning of the sequence. For example, when considering polypeptide sequences, homologues include polypeptides having substitution of one amino acid at a given position in the sequence for another amino acid of the same class (e.g., amino acids that share characteristics of hydrophobicity, charge, pK or other conformational or chemical properties, e.g., valine for leucine, arginine for lysine, etc.). Homologues can include one or more substitutions, deletions, or insertions, located at positions of the sequence that do not alter the conformation or folding of a polypeptide to the extent that the biological activity of the polypeptide is destroyed. Examples of possible homologues include polypeptide sequences and nucleic acids encoding polypeptide sequences that include substitution of one non-polar (hydrophobic) residue such as isoleucine, valine, leucine or methionine for another; the substitution of one polar (hydrophilic) residue for another such as between arginine and lysine, between glutamine and asparagine, or between threonine and serine; the substitution of one basic residue such as lysine, arginine or histidine for another; the substitution of one acidic residue, such as aspartic acid or glutamic acid for the another; or the use of a chemically derivatized residue in place of a non-derivatized residue, as long as the homolog displays substantially similar biological activity to the reference sequence.

In some embodiment, a detection nanoparticle can also include one or more detectable entities, in which case a detection regime can include detection of the multi-nanoparticle according to a size detection regime in addition to utilization of the detectable entity. As used herein, "detectable entity" is an entity that exhibits a detectable signal, e.g., an optically detectable signal, or can be modified to exhibit an optically detectable signal in the ultra-violet, visible, or near infrared electromagnetic spectrum.

In one embodiment, the detectable entity can be a material as may be a component of the nanoparticle itself, e.g., a metal of a synthetic detection nanoparticle. In other embodiments, a detectable entity can be attached to a detection nanoparticle either directly or indirectly either prior to, in conjunction with, or following attachment of the binding agent(s). A detectable entity can be attached to a detection nanoparticle either directly or indirectly using a linking agent, examples of which are described above with regard to the binding agents. For instance, a detectable entity can be attached to a surface of the detection nanoparticle or to another component at a surface of the detection nanoparticle, e.g., to a coat protein or a fusion coat protein of a phage detection nanoparticle. In some embodiments, a detectable entity can be attached to a binding agent of a detection nanoparticle, e.g., either directly to a binding agent, or indirectly by use of a linker.

In some embodiments, a detection entity can be a fluorescent entity, which can be a fluorescent protein or non-proteinaceous fluorescent material. By way of example, fluorescent detection entities can include, without limitation, proteins such as phycobiliproteins (e.g., green fluorescent protein, yellow fluorescent protein, etc.), polymers such as polyfluorenes, small organic molecule dyes such as xanthenes (e.g., fluorescein or rhodamines), cyanines, oxazines, coumarins, acridines, oxadiazoles, pyrenes, pyrromethenes, or metallo-organic complexes, such as Ru, Eu, Pt complexes. In addition to single molecule entities, clusters of fluorescent proteins or small organic molecule dyes, as well as nanoparticles, such as quantum dots, upconverting nanoparticles, gold nanoparticles, dyed polymer nanoparticles can also be used as fluorescent moieties.

Another group of photoluminescent detection entities as may be included are phosphorescent moieties with time-delayed emission of light after excitation. Phosphorescent moieties include metallo-organic complexes, such as Pd, Pt, Tb, Eu complexes, or phosphorescent pigments as may be incorporated in a detection nanoparticle such as lanthanide doped SrAl₂O₄.

In one embodiment, the detection moiety can be a radioactive label. A radioactive label may be in the form of radioisotope labeling by exchanging nonradioactive isotopes for their radioactive counterparts, such as tritium, ³²P, ³⁵S or ¹⁴C, or introducing covalently bound labels, such as ¹²⁵I, which is bound to tyrosine, ¹⁸F within fluorodeoxyglucose, or metallo-organic complexes, i.e., ⁹⁹Tc-DTPA.

In some embodiments, a detectable entity can be detectable in the presence of an activating agent, for instance an agent capable of causing chemiluminescence by the detectable entity, e.g., horseradish peroxidase in the presence of luminol. In yet another embodiment, a detectable entity can be an energy absorbing entity, upon which the entity can emit a detectable signal. For instance, upon irradiation by a pulsed laser light, a detectable entity can generate a detectable photoacoustic signal.

To detect a BNP of interest, a detection nanoparticle can be combined with a test sample suspected of containing the BNP. A test sample may be derived from any biological source, such as a physiological fluid, including blood, interstitial fluid, saliva, ocular lens fluid, cerebral spinal fluid, sweat, urine, milk, ascites fluid, mucous, nasal fluid, sputum, synovial fluid, peritoneal fluid, vaginal fluid, menses, amniotic fluid, semen, and so forth. In addition to physiological fluids, other liquid samples may be used such as water, food products, and so forth, for the performance of environmental or food production examination. Further, a solid material suspected of containing the BNP may be used as the test sample. A test sample may be used directly as obtained from a biological source or following a pre-treatment to modify the character of the sample. For example, such pre-treatment may include preparing plasma from blood, diluting viscous fluids, and so forth. Methods of pre-treatment may also involve filtration, precipitation, dilution, distillation, mixing, concentration, inactivation of interfering components, the addition of reagents, lysing, etc. Moreover, it may also be beneficial to modify a solid test sample to form a liquid medium

BNPs as may be detected according to disclosed methods can include any BNP of interest. In general, a targeted BNP can have an average size of about 50 nm or greater, such as about 75 nm or greater or about 100 nm or greater, and about 300 nm or less, such as about 250 nm or less, or about 200 nm or less, in some embodiments. A targeted BNP can be a pathogenic BNP, but this is not a requirement of the disclosed systems, and non-pathogenic BNPs, as well as BNPs produced from pathogens or non-pathogens, can be detected according to disclosed methods. By way of example, a targeted BNP can include, without limitation, a virus, a bacteriophage or other small bacterial agent, a viroid, an exosome, or a whole cell, in some cases. Examples of viral pathogens encompassed herein can include, without limitation, coronavirus, influenza, HIV, HCV, HBV, HPV, dengue, Chikungunya, and West Nile. In one embodiment, a targeted viral BNP can be a SARS coronavirus particle including, without limitation, SARS (e.g., SARS-CoV-1, SARS-CoV-2), MERS, HKU (e.g., HKU1), NL63, OC43, and/or 229E. A typical SARS-CoV-2 viral particle is approximately 100 nm in diameter. Upon binding with a detection nanoparticle, the multi-nanoparticle complex thus formed can thus be about 150 nm or even greater.

In one embodiment, a larger complex can be formed including multiple targeted BNPs and optionally also including multiple detection nanoparticles bonded in a larger, three-dimensional complex. The larger multi-nanoparticle complex can then be detected by the larger size of the complex and, in some embodiments, can be counted so as to provide additional information about the test sample. For instance, a multi-nanoparticle complex has a dimension of from about 200 nm to about 3 micrometers, such as about 200 nm or greater, about 300 nm or greater, or about 400 nm or greater, and/or such as up to about 1 micrometer, up to about 2 micrometers, or up to about 3 micrometers in some embodiments.

A BNP of interest can exhibit a binding partner of a specific binding agent carried on a detection nanoparticle. Many viruses that infect mammalian cells are enveloped viruses, meaning that the viral particle is enclosed in a phospholipid bilayer. This bilayer generally incorporates several different types of proteins, including structural proteins, and the viral coat protein that is generally involved is an attachment of the virus to the cell and allows subsequent entry into the cell. Many copies of the coat protein are maintained on the surface of the virus, and as such, can provide a targeted binding agent for a binding partner carried by a detection nanoparticle. For instance, in the case of a SARS-CoV-2 BNP, the BNP exhibits the SPIKE protein as well as other structural proteins that can be targeted for binding by a detection nanoparticle.

In some embodiments, a targeted binding partner of a BNP of interest can be involved in infection or other pathogenic action of the BNP. As such, complex formation between the BNP of interest and the detection nanoparticle can provide further evidence that the detection regime is targeting fully functional BNP. Examples of viral surface proteins involved in an infection by one, multiple, or all known SARS proteins as may targeted by a detection nanoparticle can include, without limitation, human coronavirus 229E surface glycoprotein (accession no. NP_073551.1), human coronavirus NL63 S protein (accession no. AFV53148.1), human coronavirus HKU1 spike glycoprotein (accession no. YP_173238.1), human coronavirus OC43 S protein (accession no. QDH43726.1), Middle East respiratory syndrome-related (MERS) coronavirus S protein (QFQ59587.1), SARS coronavirus Urbani S protein (accession no. AAP 13441.1), and severe acute respiratory syndrome coronavirus surface glycoprotein (accession no.
2YP_009724390.1).

Of course, BNP as may be detected according to disclosed methods are not limited to viral particles and other BNP of interest can be detected. By way of example, in one embodiment, exosomes of interest can be detected.

Exosomes are lipid membrane-derived vesicles, approximately 30-100 nm in size, that are secreted by most cell types. As exosomes are released by many cell types, they can be found in most body fluids, including urine, saliva, amniotic fluid, semen, breast milk, plasma, mucus, and blood. They hold a great deal of promise in disease diagnostics and drug delivery, among other uses, as they have been shown to display the same protein biomarkers as their originating cell. These biomarkers can serve as protein "fingerprints" that can herald the presence of diseased cells within the body (e.g., cancer cells, etc.), potentially long before disease symptoms arise.

Accordingly, in one embodiment, a BNP as may be detected according to disclosed methods can be an exosome, e.g., an exosome secreted from a cancer cell. In such an embodiment, a binding partner of a detection nanoparticle binding agent can include a surface or other biomarker of a cancer cell and/or a tumor marker of the cancer type.

Of course, one of skill in the art will understand that BNP as may be detected according to disclosed methods are not limited to viral particles and exosomes, and other BNP are encompassed herein including, without limitation, bacteria (e.g., bacteriophage) and viroids.

Upon combination of a detection nanoparticle and a test sample, a multi-nanoparticle complex can be formed including one or more detection nanoparticles bound to one or more BNPs of the test sample. The binding can be specific and can include covalent bonds, hydrogen bonds, immunological binding, Van der Waals forces, ionic forces, and/or other types of binding. A complex can include multiple nanoparticles, including multiple detection nanoparticles, which can be the same or different from one another, and can be bonded to multiple BNPs, which can be the same or different from one another. As mentioned previously, due to the capability of formation of multivalent detection nanoparticles combined with multivalency of many BNPs of interest, large, three-dimensional complexes can be formed in some embodiments.

A multi-nanoparticle complex formed including one or more detection nanoparticles and one or more BNP can be detected by the increase in size of the complex as compared to any single nanoparticle of the system. In general, any method of detection and discrimination of nanoparticle size can be utilized. By way of example, size-based nanoparticle detection methods and devices as may be utilized can include those that have been previously described, for instance, in US Patent No. 10,234,370 to Kato et al.; US Patent No. 9,645,070 to Stramski et al.; US Patent Application Publication No. 2007/0229823 to Sung et al.; US Patent No. 10,794,808 to Clayton et al.; all of which are incorporated herein by reference in their entirety.

In some embodiments, a size-based detection regime can be based upon the mobility of a particle in a dispersion medium. Such techniques can include Brownian motion particle dispersion approaches and nanoparticle tracker analysis approaches. Particle mobility-based approaches are premised upon the fact that the root-mean-square value of the displacement of a particle in a dispersion medium is proportional to k_{B}T/3πηd, where k_{B} is the Boltzmann constant, T is the absolute temperature, η is the coefficient of viscosity of the dispersion medium, and d is the particle size.

Nanoparticle tracker analysis (NTA), and the technique on which it is based, Particle Tracking Velocimetry (PTV), calculate particle size based on their trajectories in space. Briefly, multiple calculated particle trajectories are used to calculate the averaged mean squared displacement (MSD) curve, which is used to determine the diffusion coefficient. This is known as a LaGrangian approach.

Particle displacement measurement techniques based upon Brownian motion analyze particles in a continuum. In these techniques, individual trajectories are not calculated, but rather, correlation is used to determine the difference in the displacement of many particles over a time period. This is known as an Eulerian approach.

In one embodiment, a particle displacement detection regime can include capturing images of the particles as they disperse in the medium at prescribed time intervals Δt (e.g., via a pulsed laser and coordinated camera). The images can then be analyzed by use of a computing system or the like to determine the displacement and thus the particle size. For instance, a method can include obtaining and recording at least first and second images of a sample including the multi-nanoparticle complexes in a dispersion fluid, wherein a first image is obtained at a first time (ti) and the second image is subsequently obtained at a second time (t₂), determining the average displacement of the particles in an area of the first and second images during a time period (At) between the first time (ti) and the second time (t₂) based on the first and second images, and then determining a diffusion coefficient of the particles in the area of the first and second images based on the average displacement of the particles during the time period (At).

According to one embodiment, a method can include obtaining and recording a series of images of the sample over a period of time, partitioning each of the series of images into interrogation areas, determining the average displacement of the particles in each of the interrogation areas in each of the series of images over the time period, determining a diffusion coefficient of the particles in each of the interrogation areas in each of the series of images based on the average displacement of the particles, and then determining an average diffusion coefficient of the particles by averaging the diffusion coefficients in each of the interrogation areas in each of the series of images. In some embodiments, the dispersion medium may be allowed to flow along a known axis, e.g., along a lateral flow device, in which case the particle displacement can be determined by subtracting a moving component of the fluid due to the known flow velocity of the medium from the total dispersion of the imaged particle, thereby determining the net particle displacement value in the flowing system. Any combination of directions of fluid motion and particle motion can be included in an analysis, e.g., vertical motion of a particle in conjunction with horizontal motion of a fluid to account for large density difference between the fluid and the particles, to obtain the particle size.

In some embodiments, a lateral flow device may be used that includes a matrix that separates particles based on size such that particles of a larger size may only move to a certain point after which they are stuck in the matrix while smaller particles continue to move through the device. One nonlimiting example of such a matrix is a polyacrylamide gel of a predetermined percentage or in a continuous or discontinuous gradient of porosity. A similar approach can be used to separate the particles using filtration through different sized pores.

In some embodiments, a size-based detection regime can include determination of a particle size distribution, which can provide further information about the complex formation of a system, e.g., proportion of multi-nanoparticle complexes that include only 2 particles, 3, particles, 5 particles, etc. In addition, the number of multi-nanoparticle complexes imaged in a regime can be counted, which can provide additional information about a sample, and in particular, can provide a concentration of the BNP of interest in the sample.

Detection approaches are not limited to particle mobility-based approaches, however, and other size-based detection regimes can be utilized including, without limitation, dark field microscopy and transmission electron microscopy (TEM).

Briefly, a dark-field microscopy approach includes measuring a scattering intensity of particles in a sample with a dark-field microscope and correlating a brightness of the particles to a particle size distribution of the particles in the sample. A reference sample may be used to determine the correlation between the brightness of the particles and the size of the particles. The brightness and location of individual particles within the field of view of the dark-field microscope can be recorded, for example, by a digital camera. The concentration of particles can then be determined by counting the number of particles in a given suspension volume. The particle size distribution can be constructed based on the relative brightness (scattering intensity) of individual particles after the instrument is calibrated with reference particles prior to the measurement of the sample suspension. Alternatively, the reference particles can be added to the sample suspension for calibration. In addition or alternatively, the average diffusion coefficient of the particles (and their average hydrodynamic diameter) can be determined by tracking the distance traveled by individual particles over a period of time.

Disclosed methods can be beneficially utilized in diagnostic applications, e.g., diagnosis of the presence of a disease state such as a viral infection or a cancer, either prior to or following onset of symptoms, as well as in public health applications, e.g., examination of wastewater or other environmental sources for presence of intact, functional BNP.

### Example 1

A bacteriophage was formed to display a single scFv, scFV CR3022 (CR3022 Heavy Chain (Accession #ABA54613), Light Chain (Accession #ABA54614)). The heavy and light chains of the individual scFv are linked via the sequence GGGGSGGGGSGGGGS (SEQ ID NO: 1). This scFv has been previously identified as an antibody against the Spike protein of SARS-CoV-1 and has been shown to bind to the spike protein of SARS-CoV-2.

DNA encoding the scFv was engineered such that the DNA sequence was attached to the 3' end of DNA encoding the lambda phage gpD protein with a short piece of DNA in between which codes for the amino acid sequence, GGSGPVGPGGSGAS (SEQ ID NO: 2).

The engineered protein was expressed in bacteria simultaneously with lambda infection of the same bacterium. These bacteria produced new lambda phage which incorporate the gpD-linker-scFv fusion protein together with natural phage gpD. The complete sequence for CR3022 is:
SEQ ID NO: 3 
1 MTSKETFTHYQPQGNSDPAHTATAPGGLSAKAPAMTPLMLDTSSRKLVAW 50
51 DGTTDGAAVGILAVAADQTSTTLTFYKSGTFRYEDVLWPEAASDETKKRT 100
101 AFAGTAISIVGGSGPVGPGGSGASQMQLVQSGTEVKKPGESLKISCKGSG 150
151 YGFITYWIGWVRQMPGKGLEWMGIIYPGDSETRYSPSFQGQVTISADKSI 200
201 NTAYLQWSSLKASDTAIYYCAGGSGISTPMDVWGQGTTVTVSSGGGGSGG 250
251 GGSGGGGSDIQLTQSPDSLAVSLGERATINCKSSQSVLYSSINKNYLAWY 300
301 QQKPGQPPKLLIYWASTRESGVPDRFSGSGSGTDFTLTISSLQAEDVAVY 350
351 YCQQYYSTPYTFGQGTKVEIK 371
 where amino acids 1-110 encode for gpD, amino acids 111-124 (italic) encode for a linker (SEQ ID NO: 2, amino acids 125-243 (bold) encode for the heavy chain variable region of CR3022, amino acids 244-258 encode for a linker (SEQ ID NO: 1) and amino acids 259-371 encode for the light chain variable region of CR3022.

The nucleic acid sequence encoding the fusion protein gpD-CR3022 was determined from the amino acid sequence and was optimized for codon usage in E. *coli* K12. The nucleic acid sequence for the gpD-CR3022 fusion protein was cloned into the expression vector pACYCDuet-1 (Novagen^{®}) in the first multi-cloning site at the restriction site for Ncol. The resultant plasmid was sequenced across all junctions to ensure proper construction. Expression was under a T7 promoter. The final vector, designated pACYC-CR3022, was transfected into E. *coli* BL21 (DE3). Resultant phage produced in E. *coli* expressing the gpD-CR3022 represent super-antibodies and were designated ATHR-B6.

Expression of ATHR-B6 was achieved as follows: *E. coli* transfected with the pACYC-CR3022 expression vector were selected on chloramphenicol containing medium, grown to an OD₆₀₀ = 0.4-0.8 at 37°C, induced with IPTG and simultaneously infected with wildtype bacteriophage λ (ATCC 23724 B2) at an MOI of 1-10. Cultures were allowed to grow for 4-5 hours.

Phage were isolated by centrifugation of cultures at 10000xg to remove cells and cell debris. The supernatant was clarified through a 0.45 µ filter and then a 0.22 µ filter and then concentrated -25-fold and buffer exchanged into PBS using tangential flow filtration (TFF) through a Minimate^{™} TFF Capsule 500k Omega^{™} Membrane (Pall).

ATHR-B6 were biochemically characterized. The particle formation was established using the NanoSight NS300 Instrument (Malvern Panalytical).

Briefly, streptavidin-488 (SA-488) was used as a fluorescent marker. SA-488 bonded to biotinylated Spike RBD protein (SRBD-b), which in turn is bonded by the CR3022 antibody. A solution was formed including 30 µL CR3022, 10 µL SRBD-b, and 20 µL SA-488. The solution was set on ice for 30 minutes before diluting by 1/500 with H₂O and a fluorescent filter on the NS300 was used to detect fluorescence and confirm formation.

Dot blots (FIG. 2), Western blots and sandwich ELISAs with antibodies against human antibody sequences were used to establish expression of the scFv on the surface of the phage. The functionality of the displayed scFv was demonstrated by binding to recombinant Spike-RBD protein by ELISA.

On the dot blot images of FIG. 2, the columns were as follows:
1) A: CR3022 (Example 1)
2) B: ACE2 (Example 2)
3) B2: ACE2 grown at higher temperature
4) 10 µL Rabbit Anti-Human IgG added to #1, 10 µL Rabbit Anti-ACE2 added to #2
5) After an hour on orbital shaker, washing, and blocking with milk, added 10 µL Goat Anti-Rabbit IgG-AP
6) After an hour on shaker, washed and added substrate before it went back on shaker to develop

### Example 2

A sequence covering amino acids 19-605 of the natural human ACE2 protein (Accession #BAB40370) were included in a fusion protein of an engineered bacteriophage.

DNA encoding a fusion protein was engineered such that the DNA sequence was attached to the 3' end of DNA encoding the lambda phage gpD protein with a short piece of DNA in between which codes for the amino acid sequence, GGSGPVGPGGSGAS (SEQ ID NO:2). The engineered protein was expressed in bacteria simultaneously with lambda infection of the same bacterium. These bacteria produced new lambda phage which incorporated the gpD-linker-ACE2 protein together with natural phage gpD. The sequence of the expressed protein is shown below:
SEQ ID NO: 4 
1 MTSKETFTHYQPQGNSDPAHTATAPGGLSAKAPAMTPLMLDTSSRKLVAW 50
51 DGTTDGAAVGILAVAADQTSTTLTFYKSGTFRYEDVLWPEAASDETKKRT 100
101 AFAGTAISIVGGSGPVGPGGSGASSTIEEQAKTFLDKFNHEAEDLFYQSS 150
151 LASWNYNTNITEENVQNMNNAGDKWSAFLKEQSTLAQMYPLQEIQNLTVK 200
201 LQLQALQQNGSSVLSEDKSKRLNTILNTMSTIYSTGKVCNPDNPQECLLL 250
251 EPGLNEIMANSLDYNERLWAWESWRSEVGKQLRPLYEEYVVLKNEMARAN 300
301 HYEDYGDYWRGDYEVNGVDGYDYSRGQLIEDVEHTFEEIKPLYEHLHAYV 350
351 RAKLMNAYPSYISPIGCLPAHLLGDMWGRFWTNLYSLTVPFGQKPNIDVT 400
401 DAMVDQAWDAQRIFKEAEKFFVSVGLPNMTQGFWENSMLTDPGNVQKAVC 450
451 HPTAWDLGKGDFRILMCTKVTMDDFLTAHHEMGHIQYDMAYAAQPFLLRN 500
501 GANEGFHEAVGEIMSLSAATPKHLKSIGLLSPDFQEDNETEINFLLKQAL 550
551 TIVGTLPFTYMLEKWRWMVFKGEIPKDQWMKKWWEMKREIVGVVEPVPHD 600
601 ETYCDPASLFHVSNDYSFIRYYTRTLYQFQFQEALCQAAKHEGPLHKCDI 650
651 SNSTEAGQKLFNMLRLGKSEPWTLALENVVGAKNMNVRPLLNYFEPLFTW 700
701 LKDQNKNSFVG 711
 where amino acids 1-110 encode for gpD, amino acids 111-124 (italic) encode for a linker (SEQ ID NO: 2), amino acids 125-711 (bold) encode for the ACE2 (19-605).

The nucleic acid sequence for the gpD-ACE2 fusion protein was cloned into the expression vector pACYCDuet-1 (Novagen^{®}) in the first multi-cloning site at the restriction site for Ncol. The resultant plasmid was sequenced across all junctions to ensure proper construction. Expression was under a T7 promoter. The final vector, designated pACYC-ACE2, was transfected into E. *coli* BL21 (DE3). Resultant phage produced in E. *coli* expressing the gpD-ACE2 represent super-binders and are designated ATHR-B1. This produced a multivalent receptor mimic with potentially greater affinity/avidity for SARS-CoV-2 over the natural cell surface ACE2 receptor.

ATHR-B1 was expressed and isolated as detailed in example 1.

ATHR-B1 were biochemically characterized. The particle formation was established using the NanoSight NS300 Instrument (Malvern Panalytical) with nanoparticle tracking analysis (NTA) software to establish the size and count of bio-nanoparticles.

Briefly, streptavidin-488 (SA-488) was used as a fluorescent marker. SA-488 bonded to biotinylated Spike RBD protein (SRBD-b), and ACE2 acts as a receptor to the SRBD-b. A solution was formed including 30 µL ACE2, 10 µL SRBD-b, and 20 µL SA-488. The solution was set on ice for 30 minutes before diluting by 1/500 with H₂O and a fluorescent filter on the NS300 was used to detect fluorescence and confirm formation.

Dot blots (FIG. 2), Western blots and sandwich ELISAs with antibodies against ACE2 were used to establish expression on the surface of the phage. The functionality of the displayed ACE2 was demonstrated by its binding to recombinant Spike-RBD protein by ELISA.

On the dot blot images of FIG. 2, the columns were as follows:
1) A: CR3022 (Example 1)
2) B: ACE2 (Example 2)
3) B2: ACE2 grown at higher temperature
4) 10 µL Rabbit Anti-Human IgG added to #1, 10 µL Rabbit Anti-ACE2 added to #2
5) After an hour on orbital shaker, washing, and blocking with milk, added 10 µL Goat Anti-Rabbit IgG-AP
6) After an hour on shaker, washed and added substrate before it went back on shaker to develop

### Example 3

The ability of the detection particles formed described in Examples 1 and 2 (CR3022 and ACE2 displaying phage) was demonstrated. The particles were utilized to detect a mimic of a SARS-CoV-2 coronavirus, which was a phage that displays the Spike receptor binding domain of SARS-CoV-2, ATHR-M3. The ATHR-M3 (M3) particle was combined with either the CR3022 displaying phage (ATHR-B6) or the ACE2 displaying phage (ATHR-B1). Solutions were formed as described in Table 1, below.

**Table 1**

| | **CR3022** | **ACE2** | **M3** |
|---|---|---|---|
| A | 3uL | - | 3uL |
| B | - | 3uL | 3uL |
| C | 3uL | - | 15uL |
| D | - | 3uL | 15uL |
| E | 15uL | - | 3uL |
| F | - | 15uL | 3uL |

All measurements were performed on a NanoSight NS300 Instrument (Malvern Panalytical) with nanoparticle tracking analysis (NTA) software to establish the size and count of the bio-nanoparticles.

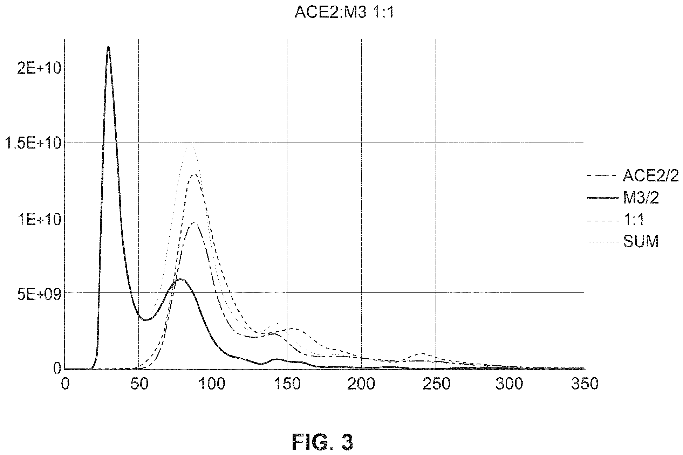

Initially, detection particles (CR3022 or ACE2) and the target BNPs (ATHR-M3) were recorded separately on the instrument and the sizes of nanoparticles were determined. Equal numbers of detection and target phage were combined and incubated for 30 minutes on ice and subsequently diluted by 1/500 with H₂O and observed on the NS300 instrument and the sizes of nanoparticles were determined. FIG. 3 displays a graph of particle count in each determination vs. particle size in nanometers. The target particles, ATHR-M3 alone, are drawn with a solid line. Prominent peaks are noted at ~30 nm and ~75 nm. ACE2 detection particles alone are drawn with a dotted and dashed line; prominent peaks at ~85 nm and 120 nm. Simple addition of the two traces, as would occur if there were no interaction of particles, results in the dotted line. The dashed line is a recording of the particles identified after incubation of equal particle numbers of both the ATHR-M3 and ACE2 BNPs. Note the disappearance of the 30 nm ATHR-M3 peak and the appearance of new peaks ~160 nm and ~240 nm, all of which indicate that an interaction between the detection and target nanoparticles has occurred.

### Example 4

To demonstrate the ability of the detection BNP, CR3022 and ACE2 displaying phage, to detect SARS-CoV-2 a mimic of a coronavirus was used, which was a phage that displays the Spike receptor binding domain of SARS-CoV-2, ATHR-M3. The complexed phage were filtered through a filtration device with a molecular cutoff that allowed the passage of single uncomplexed phage but not of larger complexes of ~100 nm in diameter. As such, complexed phage bound to each other were retained by the filter. Separated phage complexes can be detected by any number of means known to those skilled in the art for the detection of large protein structures.

## Claims

1. A method for detecting a bio-nanoparticle comprising:
contacting a test sample comprising a bio-nanoparticle having a size dimension of about 50 nanometers or greater with a detection nanoparticle, the detection nanoparticle having a size dimension of about 50 nanometers or greater, the detection nanoparticle comprising a plurality of binding agents at a surface of the nanoparticle; wherein
upon the contact, the detection nanoparticle binds a plurality of the bio-nanoparticle via a specific binding between one of the binding agents and a binding partner of each of the plurality of the bio-nanoparticles, the binding forming a multi-nanoparticle complex that includes multiple bio-nanoparticles and optionally includes multiple detection nanoparticles in a three dimensional complex; and
detecting the multi-nanoparticle complex according to a size-detection regime.

2. The method of claim 1, the detection nanoparticle comprising a modified bacteriophage that has been transformed to express an exogenous linking polypeptide at a surface of the bacteriophage, the exogenous linking polypeptide comprising a linking agent, the binding agent being attached to the bacteriophage via the linking agent.

3. The method of claim 2, wherein the exogenous linking polypeptide is a component of a fusion coat protein.

4. The method of any of the preceding claims, wherein the detection nanoparticle comprises a synthetic nanoparticle.

5. The method of any of the preceding claims, wherein the bio-nanoparticle comprises a viral particle (e.g., coronavirus, influenza, HIV, HCV, HBV, HPV, dengue, Chikungunya, or West Nile virus), a bacteria, a viroid, a cell, or an exosome, such as an exosome secreted from a cancer cell.

6. The method of any of the preceding claims, wherein the size-detection regime comprises a size exclusion device, wherein the multi-nanoparticle complex is excluded from passage through or across a matrix.

7. The method of claim 6, wherein the size exclusion device is a lateral flow device.

8. The method of claim 6 or 7, wherein the size exclusion device comprises a polyacrylamide gel.

9. The method of any of claims 6 to 8, wherein the size exclusion device is a porous device, the pores having a size such that the multi-nanoparticle complex cannot pass through the pores.

10. The method of any of the preceding claims, wherein the multi-nanoparticle complex has a dimension of about 200 nm or greater, about 300 nm or greater, or about 400 nm or greater, such as up to about 1 micrometer, up to about 2 micrometers, or up to about 3 micrometers in some embodiments.

11. The method of any one of the preceding claims, wherein the plurality of binding agents comprise at least two different binding agents, the different binding agents each specifically binding a different binding partner of the bio-nanoparticle.

12. A method for diagnosing a disease or determining a prognosis comprising:
detecting a bio-nanoparticle according to the method of any of the preceding claims; wherein
the test sample is derived from a subject; wherein
detection of the multi-nanoparticle complex is indicative of the disease or prognosis in the subject.

13. The method of claim 12, wherein the bio-nanoparticle is a pathogenic bio-nanoparticle or is produced from a pathogen or is produced by a subject in response to a disease.

14. The method of claim 12 or claim 13, wherein the test sample is derived from blood, interstitial fluid, saliva, ocular lens fluid, cerebral spinal fluid, sweat, urine, milk, ascites fluid, mucous, nasal fluid, sputum, synovial fluid, peritoneal fluid, vaginal fluid, menses, amniotic fluid, or semen.

15. The method of any of claims 12-14, wherein the disease is an infectious disease or wherein the disease is a cancer.
